(19) Europäisches Patentamt
European Patent Office
Office européen des brevets

(11) Veröffentlichungsnummer: **0 291 800**
**B1**

(12) **EUROPÄISCHE PATENTSCHRIFT**

(45) Veröffentlichungstag der Patentschrift: **31.10.90**

(51) Int. Cl.[5]: **C07C 255/13**, C07C 253/30, A01N 37/34

(21) Anmeldenummer: **88107383.7**

(22) Anmeldetag: **07.05.88**

(54) **Neue Cyanhydrin-Iodpropargylether, Verfahren zu ihrer Herstellung und ihre Verwendung als Mikrobizide.**

(30) Priorität: **19.05.87 DE 3716682**

(43) Veröffentlichungstag der Anmeldung: **23.11.88 Patentblatt 88/47**

(45) Bekanntmachung des Hinweises auf die Patenterteilung: **31.10.90 Patentblatt 90/44**

(84) Benannte Vertragsstaaten: **CH DE FR GB IT LI**

(56) Entgegenhaltungen:
**EP-A- 0 209 819**
**DE-A- 3 224 503**

(73) Patentinhaber: **BAYER AG, D-5090 Leverkusen 1 Bayerwerk(DE)**

(72) Erfinder: **Schade, Gerold, Dr., Hahnenweg 8, D-5000 Köln 80(DE)**
Erfinder: **Paulus, Wilfried, Dr., Deswatinesstrasse 90, D-4150 Krefeld(DE)**
Erfinder: **Schmitt, Hans-Georg, Dr., Am Oberend 13, D-4150 Krefeld(DE)**

**EP 0 291 800 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Cyanhydrin-Iodpropargylether, ein Verfahren zu ihrer Herstellung und ihre Verwendung als Mikrobizide im Materialschutz.

Es sind bereits Iodpropargylether, die antimikrobielle Eigenschaften aufweisen, bekannt (siehe z.B. DE-OS 3 224 503 und 3 526 789). Die mikrobiziden Eigenschaften dieser bekannten Verbindungen reichen jedoch für zahlreiche Anwendungsgebiete nicht aus. So weisen z.B. die in der DE-OS 3 224 503 beschriebenen Iodpropargylether nur gegenüber bestimmten Schadmikroben eine ausreichende Wirkung auf. Der in der DE-OS 3 526 789 beschriebene Iodpropropargylether besitzt zwar eine gute mikrobizide Wirksamkeit, ist aber trotzdem als Mikrobizid für technische Materialien, insbesondere in Anstrichfarben, nicht geeignet, da er nicht die für diesen Anwendungszweck erforderliche Auslaugbeständigkeit aufweist.

Es wurde nun gefunden, daß Iodpropargylether, die sich von bestimmten Cyanhydrinen ableiten, eine gegenüber den vorbekannten Iodpropargylethern verbesserte mikrobizide Wirkung, ein verbreitertes Wirkungsspektrum und außerdem die für die Verwendung in Anstrichfarben erforderliche hohe Auslaugbeständigkeit aufweisen.

Die Erfindung betrifft daher neue Cyanhydrin-Iodpropargylether der Formel

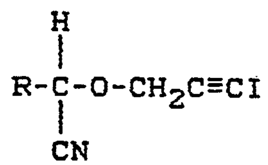

(I) ,

in der

R $C_1$-$C_{10}$-Alkyl, $C_2$-$C_{10}$-Alkenyl, $C_5$-$C_8$-Cycloalkyl oder -Cycloalkenyl, die gegebenenfalls durch $C_1$-$C_4$-Alkyl oder Halogen substituiert sein können, bedeutet.

Als Alkylreste kommen bevorzugt solche mit 2 bis 7 Kohlenstoffatomen, als Alkenylreste solche mit 2 bis 5 Kohlenstoffatomen, als Cycloalkylreste solche mit 6 bis 7 Kohlenstoffatomen und als Cycloalkenylreste solche mit 6 bis 7 Kohlenstoffatomen in Frage, wobei die Cycloalkyl- und die Cycloalkenylreste gegebenenfalls durch Methyl, Ethyl, Propyl, Chlor oder Brom substituiert sein können. Genannt werden z.B.: Ethyl, Propyl, Isopropyl, Butyl, tert.-Butyl, 1-Ethyl-pentyl, Cyclohexyl, Methylcyclohexyl, Cyclohexenyl, Methyl-cyclohexenyl, bevorzugt Ethyl, Propyl und Cyclohexenyl, ganz besonders bevorzugt Cyclohexenyl.

Die Erfindung betrifft weiterhin ein Verfahren zur Herstellung der Cyanhydrin-Iodpropargylether der Formel

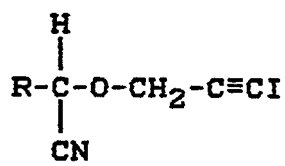

(I),

in der

R $C_1$-$C_{10}$-Alkyl, $C_2$-$C_{10}$-Alkenyl, $C_5$-$C_8$-Cycloalkyl oder -Cycloalkenyl, die gegebenenfalls durch $C_1$-$C_4$-Alkyl oder Halogen substituiert sein können, bedeutet,

das dadurch gekennzeichnet ist, daß man Cyanhydrin-Propargylether der Formel II

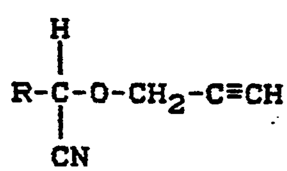

(II),

in der R die obengenannte Bedeutung hat,

mit Iodierungsmitteln in Gegenwart von Lösungs- und/oder Verdünnungsmitteln und in Gegenwart von Basen bei Temperaturen von -10°C bis +30°C umsetzt.

Als Iodierungsmittel können im erfindungsgemäßen Verfahren Iod und/oder Iodidionen liefernde Verbindungen, wie Natriumiodid und Ammoniumiodid, in Gegenwart von Oxidationsmitteln, wie Natriumhypochlorit, Calciumhypochlorit und/oder Wasserstoffperoxid, eingesetzt werden.

Als Basen eignen sich sowohl anorganische als auch organische Basen, wie Natriumhydroxid, Calciumhydroxid, Natriummethylat, Kalium-tert.-butylat und/oder Natriumisobutylat, bevorzugt Natriumhydroxid und/oder Natriummethylat.

Geeignete Lösungsmittel für das erfindungsgemäße Verfahren sind beispielsweise Wasser oder Alkohole, wie Methanol und Ethanol, oder Gemische derselben.

Bevorzugt wird die Iodierung bei Temperaturen von -5°C bis +20°C durchgeführt.

Erfindungsgemäß setzt man etwa 1 Mol Propargylether der Formel II mit etwa 1,0 bis 1,5 Mol Iodierungsmittel, bevorzugt 1,0 bis 1,2 Mol Iodierungsmittel, ein.

Die jeweils günstigsten Mengen an Basen und an Lösungs- und/oder Verdünnungsmitteln lassen sich leicht durch Vorversuche ermitteln. Üblicherweise setzt man etwa 1 bis 5, bevorzugt 1,2 bis 3 Mol Base pro Mol Propargylether der allgemeinen Formel II und die gleiche bis fünffache, bevorzugt die doppelte bis dreifache, Gewichtsmenge an Lösungs- und/oder Verdünnungsmittel ein.

Die Propargylether der allgemeinen Formel II sind zum Teil bekannt (Synth. Commun. 7 (4), 273-281 (1977)). Sie können erhalten werden, indem man Cyanhydrine der allgemeinen Formel

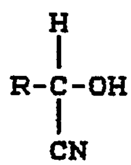 (III)

mit Propargylhalogeniden in Gegenwart von Basen umsetzt. Hierbei kann es zweckmäßig sein, die Cyanhydrine III nicht zu isolieren, sondern nur in situ herzustellen und sofort mit Propargylhalogenid weiter umzusetzen (analog zu Can. J. Chem. 55, 4200 (1977)).

Die erfindungsgemäßen neuen Cyanhydriniodpropargylether können als Wirkstoffe zur Bekämpfung von Mikroorganismen auf Pflanzen, im besonderen aber in technischen Materialien, die durch Mikroorganismen zersetzt werden können, verwendet werden.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), pflanzenschädigende Pilze sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Botrytis, wie Botrytis cinera,
Chaetomium, wie Chaetomium globosum,
Coniophora, wie Coniophora puteana,
Lentinus, wie Lentinus tigrinus,
Penicillium, wie Penicillium glaucum,
Piricularia, wie Piricularia oryzae,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylke ton, flüssige Kohlenwasserstoffe, wie Benzinfraktionen, halogenierte Kohlenwasserstoffe, wie 1,2-Dichlorethan, sein.

Mikrobizide Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, 2-Thiocyanatomethylthiobenzthiazol, Phenolderivate, wie 2-Phenylphenol, (2,2'-Dihydroxy-5,5'-dichlor)diphenylmethan und 3-Methyl-4-chlor-phenol.

Die Herstellung und die Verwendung der erfindungsgemäßen neuen Cyanhydriniodpropargylether geht aus den nachfolgenden Beispielen hervor.

Herstellungsbeispiele:

Beispiel 1

Cyclohexencarboxaldehydcyanhydrin-iodpropargylether

32,5 g KCN, 80 ml $H_2O$, 160 ml $CH_2Cl_2$ und 12,8 g Tetrabutylammoniumbromid werden vorgelegt und unter Rühren ein Gemisch aus 48 g Propargylbromid und 44 g 3-Cyclohexencarboxaldehyd zugetropft. Man rührt noch 5 h, trennt die organische Phase ab, schüttelt die wäßrige Phase dreimal mit je 100 ml $CH_2Cl_2$ aus, vereinigt die organischen Lösungen, trocknet mit $Na_2SO_4$ und engt ein. Das resultierende Öl wird destilliert, Kp. 132-6°C/15 mbar. Ausbeute 48 g Cyanhydrinpropargylether.

43,8 g davon werden in 500 ml Methanol gelöst, auf 5°C gekühlt und 37 g Natriummethylat zugesetzt. Dann gibt man 63,5 g Iod zu und rührt noch 2 h bei 0 bis 5°C. Man gießt in Eiswasser und saugt das Produkt ab. Ausbeute 69 g Cyanhydrin-iodpropargylether, Fp. 62-4°C, gelbliche Kristalle.

Analog wurden hergestellt:

| Beispiel | R der Formel (I) | Eigenschaften | IR |
|---|---|---|---|
| 2 | $CH_3CH_2-$ | Öl | 2185 $cm^{-1}$ |
| 3 | $(CH_3)_2CH-$ | Öl | 2190 $cm^{-1}$ |
| 4 | $CH_3CH_2CH_2-$ | Öl | 2187 $cm^{-1}$ |
| 5 | $(CH_3)_3C-$ | Fp. 36° C | 2189 $cm^{-1}$ |
| 6 | $CH_3(CH_2)_3CH(C_2H_5)-$ | Öl | 2182 $cm^{-1}$ |

Anwendungsbeispiele

Beispiel 7

Zum Nachweis der Wirksamkeit gegen Pilze wurden die minimalen Hemm-Konzentrationen (MHK) erfindungsgemäßer Cyanhydrin-Iodpropargylether und bekannter Iodpropargylverbindungen bestimmt:

Ein Agar, der aus Bierwürze und Pepton hergestellt wird, wird mit erfindungsgemäßen Wirkstoffen in Konzentrationen von 0,1 mg/l bis 5.000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt; sie ist in der nachstehenden Tabelle angegeben.

Tabelle I MHK's [mg/l] bei der Einwirkung verschiedener Iodpropargylether auf Pilze

| Testorganismen | verwendete Isopropargylether: erfindungsgemäße Verbindungen gemäß Beispiel 1 | 2 | 3 | Verbindungen gemäß St. d. T.: Verbindung A gemäß DE-OS 3 224 503 | Verbindung gemäß DE-OS 3 526 789 |
|---|---|---|---|---|---|
| Alternaria tenuis | 5 | 2 | 5 | 5 | 5 |
| Aspergillus niger | 0,5 | 2 | 1 | 5 | 5 |
| Aureobasidium pullulans | 5 | 5 | 2 | 10 | 5 |
| Chaetomium globosum | 5 | 3,5 | 10 | 10 | 20 |
| Cladosporium cladosporioides | 0,5 | | 1 | - | 5 |
| Lentinus tigrinus | 7,5 | 2 | 5 | 5 | 15 |
| Penicillium glaucum | 1 | 2 | 5 | 5 | 5 |
| Polyporus versicolor | | 1 | | 5 | - |
| Sclerophoma pityophila | 5 | 2 | 1,5 | 10 | 5 |

Beispiel 8

Wirkung gegen Bakterien

Ein Agar, der als Nährmedium Bouillon enthält, wird mit erfindungsgemäßen Wirkstoffen in Konzentrationen von 1 bis 5.000 ppm versetzt. Darauf infiziert man das Nährmedium jeweils mit den in Tabelle II aufgeführten Testorganismen und hält das infizierte Medium 2 Wochen bei 28°C und 60 bis 70 % rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle II wiedergegeben.

Tabelle II

Angabe der MHK-Werte in mg/l bei der Einwirkung der unten angegebenen Wirkstoffe auf Bakterien.

| Testorganismen | MHK in mg/l der Wirkstoffe Verbindung des Beispiels Nr. | | | | |
|---|---|---|---|---|---|
| | 1 | 2 | 3 | 4 | 5 |
| Escherichia coli | 750 | 100 | 100 | 100 | 500 |
| Staphylococcus aureus | 50 | 100 | 100 | 100 | 200 |

Beispiel 9 (Wirkung gegen Schleimorganismen)

Die zu testenden Iodpropargylether werden, in wenig Aceton gelöst, in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, zur Anwendung gebracht. Zu Beginn des Testes werden die einzelnen Nährlösungen mit Schleimorganismen (ca. $10^6$ Keime/ml), die aus bei der Polyamid-Herstellung verwendeten Spinnwasser-Kreisläufen isoliert wurden, infiziert. Nährlösungen, die den zu testenden Iodpropargylether in mindestens minimaler Hemmkonzentration (MHK) enthielten, sind nach 3-wöchiger Kultur bei Raumtemperatur noch völlig klar; die in wirkstofffreien Nährlösungen nach 3 bis 4 Tagen eintretende durch die starke Vermehrung der Mikroben hervorgerufene Schleimbildung und die damit verbundene Trübung wird in ihnen unterbunden.

Tabelle III

MHK-Wert [mg/l] der erfindungsgemäßen Cyanhydrin-Iodpropargylether bei der Einwirkung auf Schleimorganismen.

| Cyanhydrinpropargylether gemäß Beispiel | MHK [mg/l] |
|---|---|
| 1 | 15 |
| 2 | 7,5 |
| 3 | 15 |
| 4 | 15 |

Beispiel 10 (Wirkung gegen Algen)

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaeodactylum tricornutum Bohlin) wird unter Durchperlen von Luft in Allens Nährlösung (Arch. Mikrobiol. 17, 34 bis 53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben. Nach 2 Wochen ist die Nährlösung durch intensives Algenwachstum tief grün-blau gefärbt. Das Absterben der Algen nach Zugabe erfindungsgemäßer Wirkstoffe erkennt man an dem Entfärben der Nährlösung.

Tabelle IV

Algen-abtötende Konzentrationen [mg/l] der erfindungsgemäßen Cyanhydrin-Iodpropargylether

| Verbindung des Beispiels Nr. | abtötende Konzentration [mg/l] |
|---|---|
| 1 | 100 |
| 2 | 100 |
| 3 | 100 |
| 4 | 100 |

Beispiel 11 (Fungizide Wirkung in Anstrichmitteln)

Die fungizide Wirkung in Anstrichmitteln wird durch Prüfung der Schimmelfestigkeit der mit den Anstrichmitteln erhaltenen Anstriche bestimmt.

Die Prüfung wird in Anlehnung an den Report 219 der Defense Standards Laboratories Maribyrnong/Australien wie folgt durchgeführt:

Das zu prüfende Anstrichmittel wird beidseitig auf eine geeignete Unterlage gestrichen.

Um praxisnahe Ergebnisse zu erhalten wird ein Teil der Prüflinge vor dem Test auf Schimmelfestigkeit mit fließendem Wasser (24 Stunden; 20°C) ausgelaugt; ein anderer Teil wird mit einem warmen Frischluftstrom behandelt (7 Tage; 40°C).

Die so vorbereiteten Prüflinge werden auf einen Agar-Nährboden gelegt. Prüfling und Nährboden werden mit Pilzsporen kontaminiert. Nach 1- bis 3-wöchiger Lagerung bei 29 ± 1°C und 80 bis 90 % rel. Luftfeuchte wird abgemustert. Der Anstrich ist dauerhaft schimmelfest, wenn der Prüfling pilzfrei bleibt oder höchstens einen geringen Randbefall erkennen läßt.

Zur Kontamination werden Pilzsporen folgender neun Schimmelpilze verwendet, die als Anstrichzerstörer bekannt sind oder häufig auf Anstrichen angetroffen werden:

1. Alternaria tenuis
2. Aspergillus flavus
3. Aspergillus niger
4. Aspergillus ustus
5. Cladosporium herbarum
6. Paecilomyces variotii
7. Penicillium citrinum
8. Aureobasidium pullulans
9. Stachybotrys atra Corda

Eine Dispersionsfarbe auf Polyvinylacetatbasis, die als Fungizid 0,5 Gew.-%, bezogen auf Gesamtfeststoffgehalt, des in Beispiel 1 beschriebenen Cyanhydrin-Iodpropargylethers enthält, liefert bei obiger Prüfung dauerhaft schimmelfeste Anstriche.

Wird als Fungizid statt des in Beispiel 1 beschriebenen Iodpropargylethers der in der DE-OS 3 526 789 beschriebene Iodpropargylether eingesetzt, so werden auch bei Gehalten von 3 Gew.-% noch keine schimmelfesten Anstriche erhalten.

## Patentansprüche

1. Cyanhydrin-Iodpropargylether der Formel

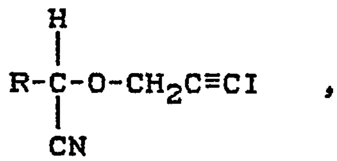

in der
R$C_1$-$C_{10}$-Alkyl, $C_2$-$C_{10}$-Alkenyl, $C_5$-$C_8$-Cycloalkyl oder -Cycloalkenyl, die gegebenenfalls durch $C_1$-$C_4$-Alkyl oder Halogen substituiet sein können, bedeutet.

2. Cyanhydrin-Iodpropargylether nach Anspruch 1, dadurch gekennzeichnet, daß
R ein Ethyl-, Propyl- oder ein gegebenenfalls durch Methyl substituierter Cyclohexenyl-Rest ist.

3. Verfahren zur Herstellung von Cyanhydrin-Iodpropargylethern der Formel

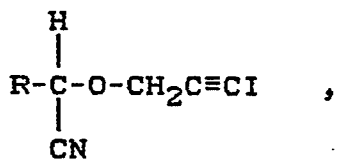

in der
R$C_1$-$C_{10}$-Alkyl, $C_2$-$C_{10}$-Alkenyl, $C_5$-$C_8$-Cycloalkyl oder -Cycloalkenyl, die gegebenenfalls durch $C_1$-$C_4$-Alkyl oder Halogen substituiert sein können, bedeutet,
dadurch gekennzeichnet, daß man Cyanhydrin-Propargylether der Formel

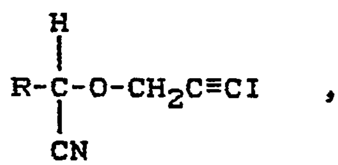

in der R die obengenannte Bedeutung hat,
mit Iodierungsmitteln in Gegenwart von Lösungs- und/oder Verdünnungsmitteln und in Gegenwart von Basen bei Temperaturen von -10°C bis +30°C umsetzt.

4. Verfahren nach Anspruch 3, dadurch gekennzeichnet, daß man als Iodierungsmittel Iod und/oder Iodidionen liefernde Verbindungen in Gegenwart von Oxidationsmitteln einsetzt.

5. Verwendung von Cyanhydrin-Iodpropargylethern der Formel

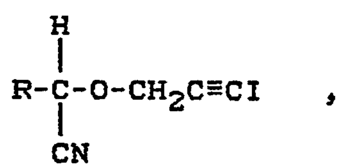

in der
R$C_1$-$C_{10}$-Alkyl, $C_2$-$C_{10}$-Alkenyl, $C_5$-$C_8$-Cycloalkyl oder -Cycloalkenyl, die gegebenenfalls durch $C_1$-$C_4$-Alkyl oder Halogen substituiert sein können, bedeutet,
als Mikrobizide zum Schutz technischer Materialien vor mikrobieller Veränderung oder Zerstörung.

6. Verwendung nach Anspruch 5 als Mikrobizid in Anstrichmaterialien und in Holzschutzmitteln.

## Claims

1. Cyanohydrin iodopropargyl ethers of the formula

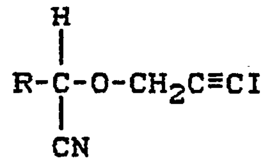

,

in which
R denotes $C_1$-$C_{10}$-alkyl, $C_2$-$C_{10}$-alkenyl, $C_5$-$C_8$-cycloalkyl or -cycloalkenyl, which can be optionally substituted by $C_1$-$C_4$-alkyl or halogen.

2. Cyanohydrin iodopropargyl ethers according to Claim 1, characterized in that R is an ethyl or propyl radical or a cyclohexenyl radical which is optionally substituted by methyl.

3. Process for the preparation of cyanohydrin iodopropargyl ethers of the formula

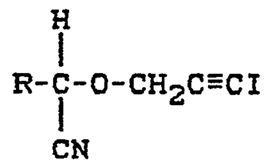

,

in which
R denotes $C_1$-$C_{10}$-alkyl, $C_2$-$C_{10}$-alkenyl, $C_5$-$C_8$-cycloalkyl ox -cycloalkenyl, which can be optionally substituted by $C_1$-$C_4$-alkyl or halogen, characterized in that cyanohydrin propargyl ethers of the formula

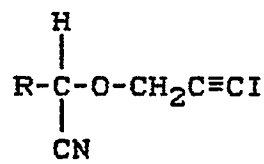

,

in which
R has the abovementioned meaning, are reacted with iodinating agents in the presence of solvents and/or diluents and in the presence of bases at temperatures of -10°C to + 30°C.

4. Process according to Claim 3, characterized in that iodine and/or compounds which supply iodide ions, in the presence of oxidizing agents, are used as the iodinating agent.

5. Use of cyanohydrin iodopropargyl ethers of the formula

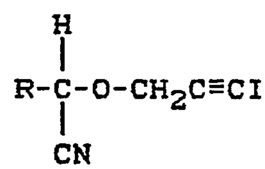

,

in which
R denotes $C_1$-$C_{10}$-alkyl, $C_2$-$C_{10}$-alkenyl, $C_5$-$C_8$-cycloalkyl or -cycloalkenyl, which can be optionally substituted by $C_1$-$C_4$-alkyl or halogen, as microbicides for preserving industrial materials from microbial change or destruction.

6. Use according to Claim 5 as a microbicide in paint materials and in wood preservatives.

## Revendications

1. Ether iodopropargylique de cyanhydrine, de formule

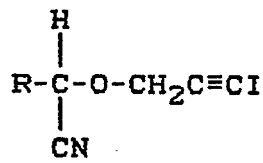 ,

dans laquelle
R est un groupe alkyle en $C_1$ à $C_{10}$, alcényle en $C_2$ à $C_{10}$, un groupe cycloalkyle ou cycloalcényle en $C_5$ à $C_8$ pouvant être substitué le cas échéant par un radical alkyle en $C_1$ à $C_4$ ou un halogène.

2. Ether iodopropargylique de cyanhydrine suivant la revendication 1, caractérisé en ce que R est un reste éthyle, un reste propyle ou un reste cyclohexényle éventuellement substitué par un radical méthyle.

3. Procédé de production d'éthers iodopropargyligues de cyanhydrines de formule

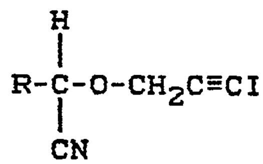 ,

dans laquelle
R est un groupe alkyle en $C_1$ à $C_{10}$, un groupe alcényle en $C_2$ à $C_{10}$, un groupe cycloalkyle ou cycloalcényle en $C_5$ à $C_8$ pouvant être substitué le cas échéant par un radical alkyle en $C_1$ à $C_4$ ou un halogène, caractérisé en ce qu'on fait réagir un éther de propargyle de cyanhydrine de formule

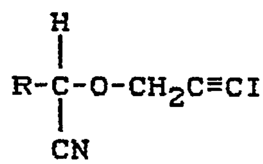 ,

dans laquelle
R a la définition indiquée ci-dessus, avec des agents d'iodation en présence de solvants et/ou de diluants et en présence de bases, à des températures de -10°C à + 30°C.

4. Procédé suivant la revendication 3, caractérisé en ce qu'on utilise comme agent d'iodation l'iode et/ou des composés engendrant des ions iodure, en présence d'agents oxydants.

5. utilisation d'éthers iodopropargyliques de cyanhydrines, de formule

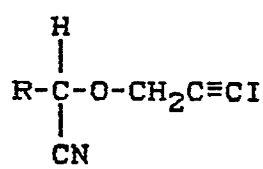 ,

dans laquelle
R est un reste alkyle en $C_1$ à $C_{10}$, alcényle en $C_2$ à $C_{10}$, un reste cycloalkyle ou cycloalcényle en $C_5$ à $C_8$ qui peut être substitué le cas échéant par un radical alkyle en $C_1$ à $C_4$ ou un halogène, comme microbicides pour protéger des matériaux industriels d'une modification ou d'une destruction microbienne.

6. utilisation suivant la revendication 5 comme microbicide dans des peintures et dans des compositions pour la protection du bois.